# EUROPEAN PATENT SPECIFICATION

(11) **EP 1 850 809 B1**
(45) Date of publication and mention of the grant of the patent: **28.07.2010**
(21) Application number: 05817951.6
(22) Date of filing: 27.12.2005
(51) Int. Cl.: A61F 2/46, A61F 2/44

(54) **A DEVICE FOR TOTAL SPINAL DISC REPLACEMENT**
VORRICHTUNG FÜR DEN BANDSCHEIBEN-TOTALERSATZ
DISPOSITIF DE REMPLACEMENT TOTAL D'UN DISQUE VERTEBRAL

(30) Priority: 28.12.2004 CH 216004
(43) Date of publication of application: 07.11.2007
(73) Proprietor: Synthes GmbH, 4436 Oberdorf (CH)
(72) Inventor: LECHMANN, Beat, CH-2544 Bettlach (CH); FRIGG, Robert, CH-2544 Bettlach (CH); PAVLOV, Paul, W., NL-6523 Njimegen (NL); BUERKI, Roger, CH-4710 Balsthal (CH); FEIGENWINTER, Gregor, CH-4432 Lampenberg (CH); APPENZELLER, Andreas, CH-2504 Biel (CH)
(74) Representative: Lusuardi, Werther
(86) International application number: PCT/CH2005/000776
(87) International publication number: WO 2006/069464

(56) References cited:
- US-A1- 2004 158 254

## Description

Today the state of the art concerning total spinal disc replacement device insertion is the anterior approach, i.e. true anterior, anterolateral (oblique) or true lateral. The surgeon decides between transperitoneal or retroperitoneal approaches, depending on the treated level or his personal experience. However, due to the presence of major vessels (e.g. aorta, vena cava) the anterior approach to the spine is often performed in the presence of vascular surgeons thereby causing extra costs.

In US 6,719,794 GERBER a method for insertion of an intervertebral fusion implant is disclosed. The removal of the affected spinal disc as well as the insertion of the implant is performed via a posterior approach offset from the midline of the spine to the affected intervertebral disc. Disadvantageously, such a transforaminal approach includes a partial dissection of the facet joint.

A prosthetic device for transforaminal insertion into an intervertebral space is known from US 2004/0225365 EISERMANN. This known prosthesis comprises two components having an articulating surface each in order to permit articulating motion between the first and second components and consequently of the adjoining vertebrae. The transforaminal path disclosed might cause potential damage to important anatomical structures such as nerve roots, dura, ligamentum flavum and interspinous ligaments.

The closest prior art is represented by US 2004/158 254.

Therefore, it is an object of the present invention to provide a method for total spinal disc replacement device insertion including a dorsal approach to the spine without having to dissect the facet joint neither completely nor partially.

It is a further object of the invention to provide an intervertebral prosthesis having a contour when viewed orthogonally to its central axis which has a minimal width but still an adequate articulation area.

According to the invention the above object is achieved through an intervertebral prosthesis which essentially comprises a first and a second prosthetic component each having an apposition surface disposed transversely to the central axis and apt for contacting the end plate of an adjoining vertebral body each. Furthermore, said first and second prosthetic components are connected by means of an articulation. When viewed parallel to said central axis said first and second prosthetic components have an elongated shape with a major axis and a transverse minor axis, whereby said central axis, major axis and transverse minor axis intersect each other and said central axis and transverse minor axis defining a middle plane. Said first and second prosthetic component have a cross-sectional area orthogonal to said central axis which is essentially oval or elliptical and said cross-sectional area comprises at least a first and a second concavity lying on different side of the middle plane and on the same side of the major axis.

The intervertebral prosthesis according to the invention offers the following specific advantages:
- the first and second prosthetic components have a small width at their portions towards their lateral ends but still an adequate width adjacent the middle plane where the articulation is located, said small width allowing an implantation by means of an extraforaminal access and said adequate width adjacent the middle plane allowing the configuration of an articulation adapted to the natural intervertebral disc;
- un-necessary material on the first and second prosthetic components (bone contact plates) is removed in order to give special attention to the fact that the bony endplates of the vertebral bodies change their shape at the location where the prosthesis is situated during their degeneration, i.e. they become more undulated over time; and
- possible osteophytes on the posterior periphery of the vertebral endplates which the surgeon decides not to remove are taken into consideration, i.e. the intervertebral prosthesis can be positioned easier because the prosthesis can be manipulated around the undulations.

In a preferred embodiment of the intervertebral prosthesis said cross-sectional area of said first and second prosthetic component is kidney shaped with an enlargement arranged essentially symmetrical to said the middle plane.

In a further embodiment said at least two concavities have an essentially semi-elliptical or semi-oval shape.

In another embodiment said at least two convavities are disposed essentially symmetrical to said middle plane.

In still another embodiment said first and second prosthetic component have a length H measured parallel to said major axis and wherein each of said at least two concavities has a width W measured parallel to said major axis, said width W amounting to between 15% and 35% of said length H.

In yet another embodiment said first and second prosthetic component have a maximum width B measured parallel to said transverse minor axis and wherein each of said at least two concavities has a depth T measured parallel to said transverse minor axis, said depth T amounting to between 5% and 25% of said maximum width B.

In a further embodiment said cross-sectional area of said first and second prosthetic components has an essentially elliptical periphery with a smaller radius of curvature at the first subsidiary vertex than at the second subsidiary vertex of said periphery.

In another embodiment said first subsidiary vertex is on the same side of said major axis as said at least two concavities.

In a further embodiment, the ratio of the length H of the prosthetic components to the width B thereof is between 3 : 1 and 5:1. The advantage of this design essentially is that in order to implant the two prosthetic components in the intervertebral space (intervertebral disk) it is only necessary to clear a narrow access path such permitting an extraforaminal apporach.

In yet a further embodiment said articulation is configured as a ball-and-socket joint comprising a spherical cap connected to one of the first and second prosthetic components and congruent to said spherical cap a spherical recess in the other of said first and second prosthetic components.

In another embodiment, the intervertebral prosthesis is realised in two pieces, i.e. the spherical cap is made in one piece with one of the two prosthetic components.

In a further embodiment, the intervertebral prosthesis is realised in three pieces, so that the spherical cap forming the third piece is connectable with one of the two prosthetic components. The advantage of this design is to be seen essentially in the fact that the plate and the spherical cap may be realised by using different materials, so that it is possible to achieve optimal sliding properties for the articular surfaces. Preferred materials for the plates are titanium or a titanium alloy as well as PEEK or coated variants, and for the spherical cap highly crosslinked polyethylene (X-UHMWPE), an alloy of cobalt and chrome, or a ceramic material.

In again a further embodiment, the intervertebral prosthesis is realised in at least three pieces and comprises an articular shell including the recess, whereby said articular shell is attachable to one of said first and second prosthetic components as a third piece. The advantage of this design is to be seen essentially in the fact that the plate and the spherical cap may be realised using different materials, so that it is possible to achieve optimal sliding properties for the articular surfaces.

In a further embodiment, the spherical cap and the articular shell consist of a material combination made of metal and plastic. The advantages of this embodiment are that it is possible to use proven combinations of joint replacement materials such as, for example, highly crosslinked polyethylene (X-UHMWPE) and an alloy of cobalt and chrome. Further advantages are to be seen in the fact that low frictional forces are achievable for the relative displacement of the articular surfaces and that a compensation of axial impact loads can be achieved.

In yet another embodiment a ceramic-to-ceramic articulation is used.

In a further embodiment the surfaces of the spherical cap and the recess are coated with titanium carbide or with amorphous carbon (ADLC) therewith permitting a substantial reduction of the coefficient of friction.

In another embodiment, at least the spherical cap is made of a memory metal or of a material capable of swelling (e.g. hydrogels).

In still another embodiment at least the spherical cap is made of a flowable, thermosetting material. The monomers, comonomers, homopolymers, oligomers, or mixtures which contain such thermosetting, flowable substances may suitably be selected from the group of:
a) polyethylene glycols, preferably polyethylene glycol(di)-acrylates;
b) n-vinylpyrrolidones; and
c) vinyls, preferably vinyl alcohols; and
d) styrenes.

The polymers thus obtained may be widely varied as regards their elasticity. The advantages of these designs are to be seen essentially in the fact that due to the reduced volume of the joint, the insertion of the intervertebral prosthesis becomes less invasive, the increased volume being best suited for achieving an optimal articular function.

In a further embodiment the first prosthetic component is selected from a first kit of at least M ≥ 2 first prosthetic components and the second prosthetic component is selected from a second kit of at least N ≥ 2 second prosthetic components. Said first and second kit may comprise first and second prosthetic components being provided with different heights, articulation radii or locations of the centre of the respective radii,
i.e. the center of rotation. By means of this embodiment the following advantages may be achieved:
   - the position of the centre of rotation is adjustable in height as the two component parts may be arranged in a modular manner;
   - the components which may also vary in the radius of the spherical cap, which makes it possible to adjust the centre of rotation of the intervertebral prosthesis;
   - the position of the centre of rotation, the angulation, and the portion of translatory motion of the intervertebral prosthesis may be freely selected within a relatively wide range, the portion of translatory motion being the portion of movement measured transversely to the central axis of the prosthetic components relative to each other;
   - the prosthetic components may also take into account the articulation requirements of the motion segment in that it is possible to include spherical caps having different radiuses. Greater spherical cap radiuses have a higher portion of translatory motion during deflection, which on the flexion of a patient's spine puts increased pressure on the facet joints and leads to an accelerated degeneration thereof;
   - the heights of the prosthetic components and the corresponding radiuses of the articulating spherical cap of the intervertebral prosthesis may vary and are thus adaptable to the dimensions of different intervertebral spaces.

In yet a further embodiment said first and second prosthetic component each comprise an intermediate surface, said first and second intermediate surfaces facing each other. At least the first or second intermediate surface is provided with at least one cavity with an undercut being located at the first or/and second lateral end of said first and second prosthetic components, said cavity with an undercut being apt for acceptance of an anchoring member of an implant holder.

Further objects and advantages of the invention will become apparent from the following description when read with reference to the accompanying drawings which illustrate the method according to the invention. In the drawings:
Fig. 1 is a cross-section through the spinal column orthogonal to the longitudinal axis of the spinal column;
Fig. 2 is a plane view to the back of a patient in prone position;
Fig. 3 is a plane view to the dorsal muscle configuration of a patient;
Fig. 4 is a cross-section through the body of a patient orthogonal to the longitudinal axis of the spinal column and seen from cranial;
Fig. 5 is a perspective view to the lumbar part of the spinal column whereby a Kirschner wire is inserted into the affected spinal disc according to one step of an implantation procedure;
Fig. 6 is a perspective view to the lumbar part of the spinal column whereby a trockar and a protection sleeve are slid over the Kirschner wire according to one step of an implantation procedure;
Fig. 7 is a perspective view to the lumbar part of the spinal column whereby a cutting device is slid through the protection sleeve according to one step of an implantation procedure;
Fig. 8 is a perspective view to the lumbar part of the spinal column whereby an access portal is being cut into the affected disc space;
Fig. 9 is a perspective view to the lumbar part of the spinal column together with a distractor according to one step of an implantation procedure;
Fig. 10 is a perspective view to the lumbar part of the spinal column together with an intervertebral prosthesis and an implant holder according to one step of an implantation procedure;
Fig. 11 is a lateral view to the portion of the spinal column together with the inserted intervertebral prosthesis;
Fig. 12 is a view from anterior to the portion of the spinal column together with the inserted intervertebral prosthesis;
Fig. 13 is a top view of an embodiment of the intervertebral prosthesis according to the invention;
Fig. 14 is a front view of the embodiment of fig. 13;
Fig. 15 is a lateral view of the embodiment of figs. 13 and 14;
Fig. 16 is a perspective view of the second prosthetic component of the embodiment of figs. 13-15;
Fig. 17 is a cross-section along the major axis of the second prosthetic component of fig. 16;
Fig. 18 is a magnified view of one lateral end of the second prosthetic component of figs. 16 and 17;
Fig. 19 is a perspective view of the second prosthetic component of figs. 16 - 18 together with the leading end of an implant holder; and
Fig. 20 is a perspective view of the second prosthetic component of figs. 16 - 18 together with a portion of the sleeve of an implant holder.

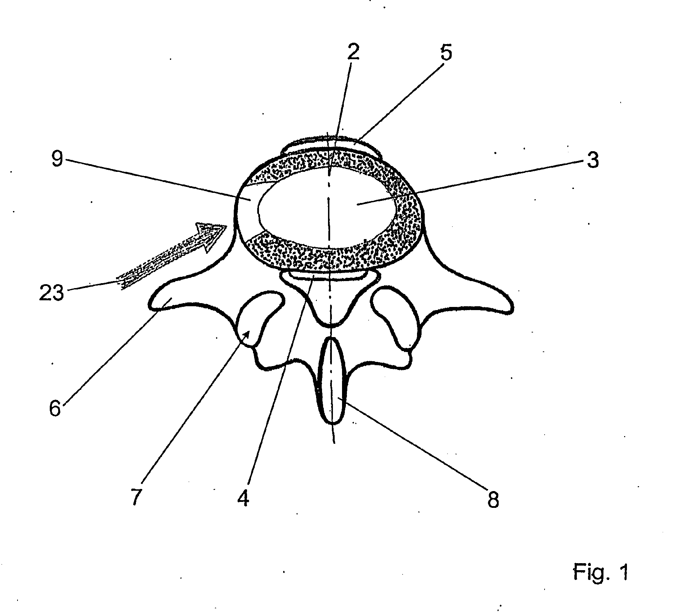

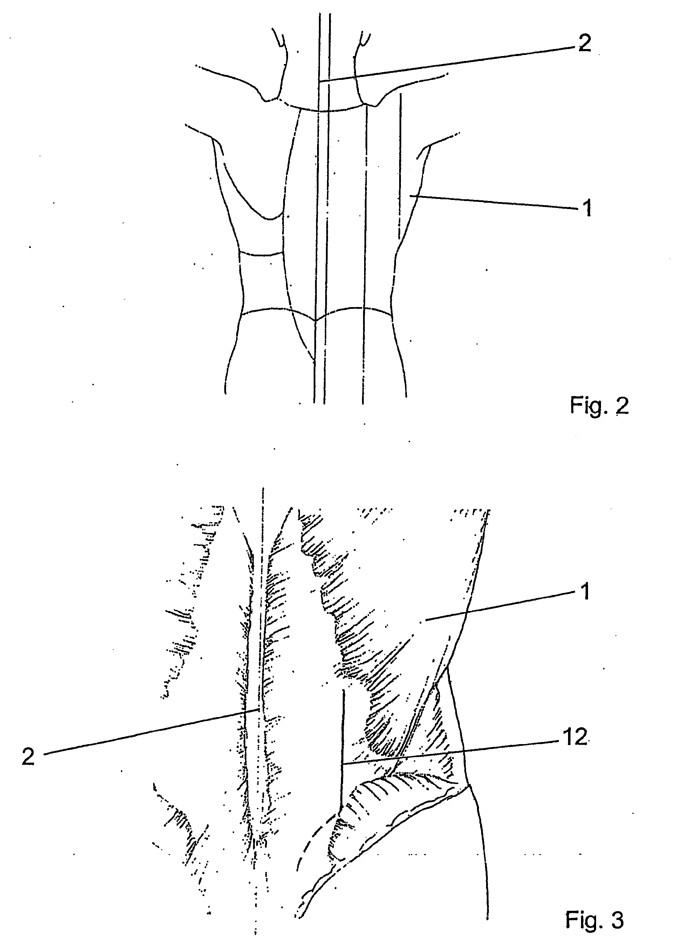

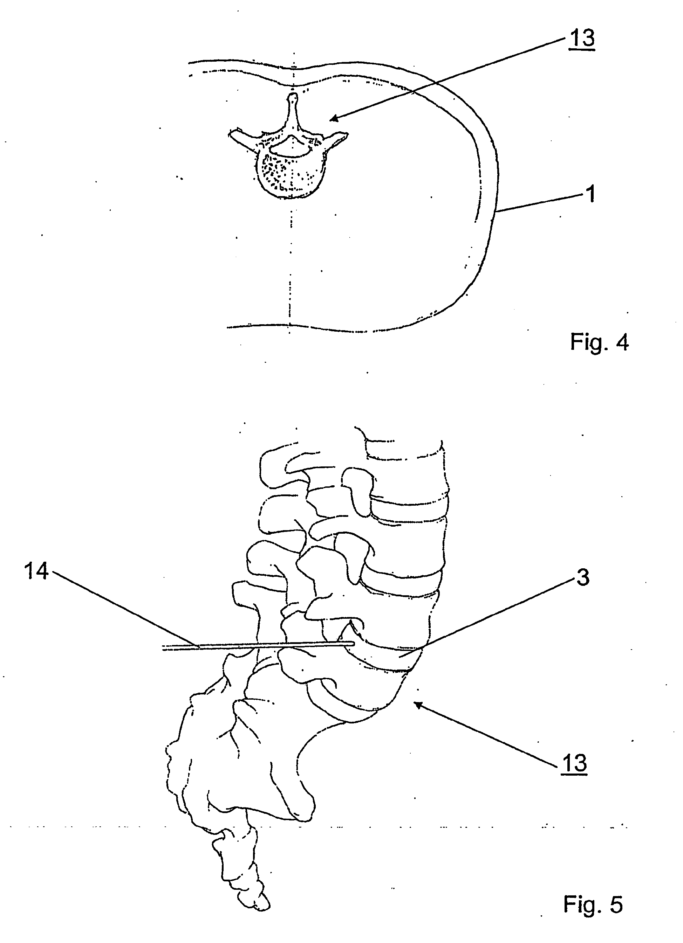

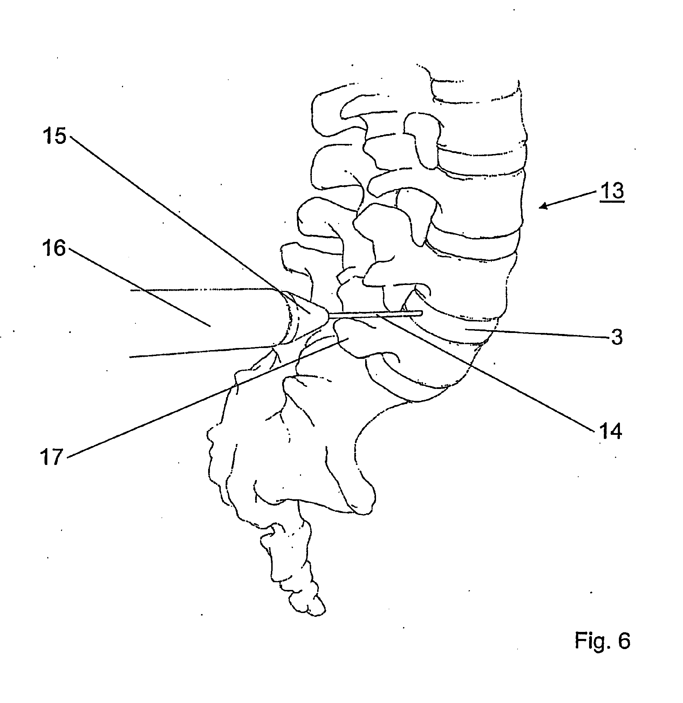

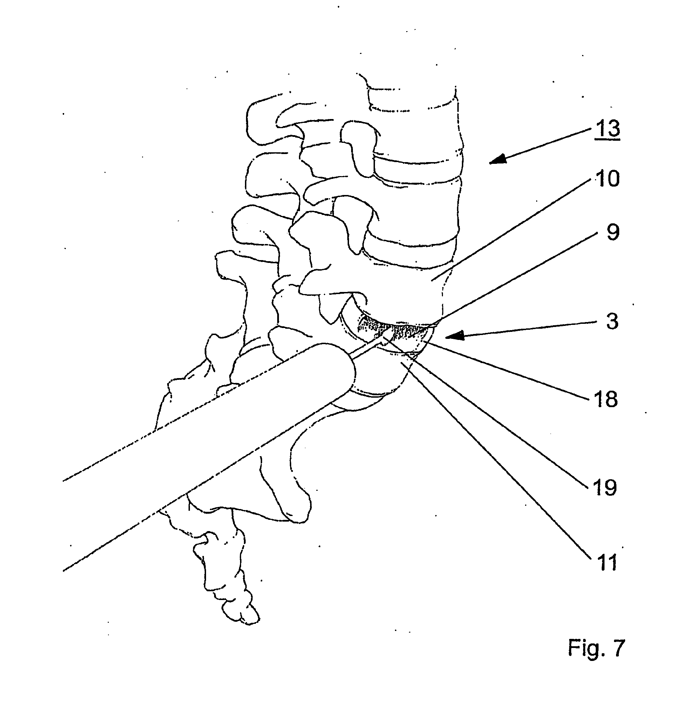

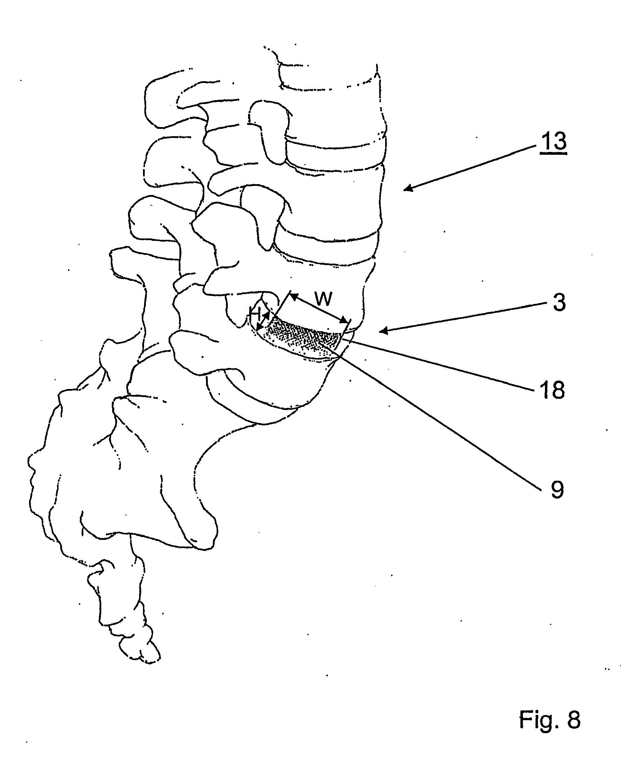

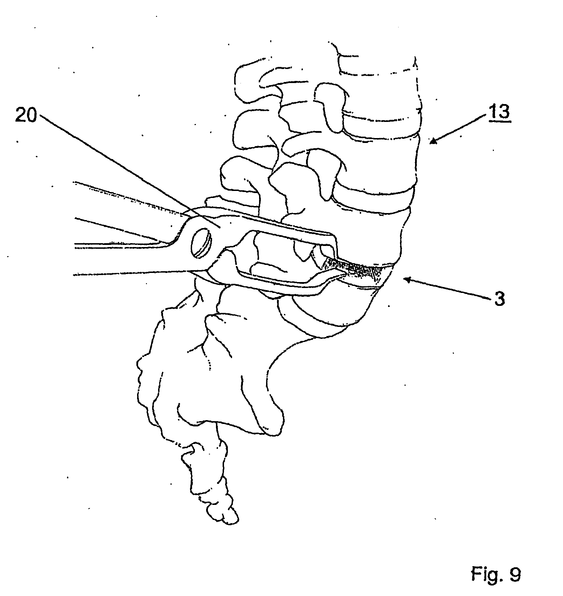

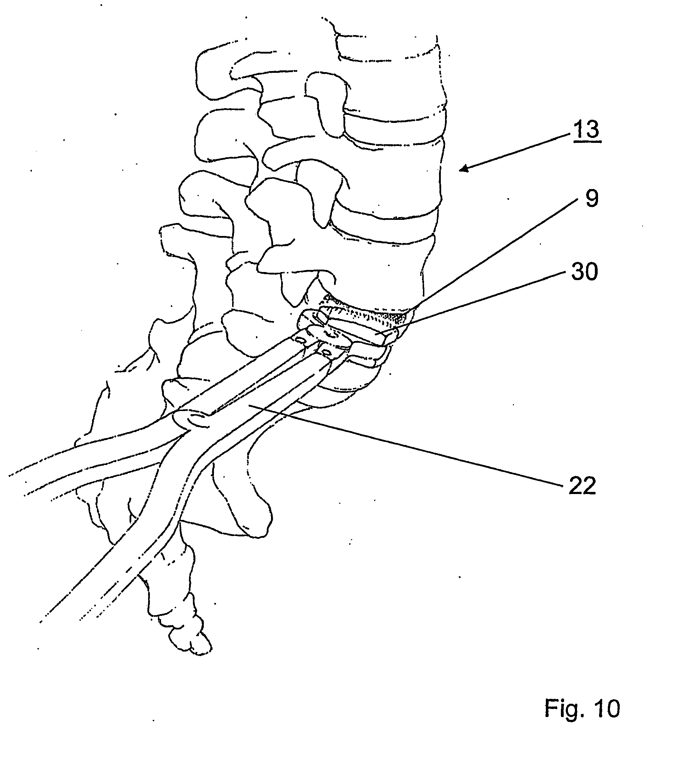

Description of the method for replacement of a spinal disc through an extraforaminal approach:
1. Performing a dorsal incision 12 approximately 7 cm extra-medial, i.e. apart from the median longitudinal plane 2 whereby the Fascia Lumbodorsalis is targeted in order to minimize the muscular destruction (Fig. 3);
2. Approaching the affected intervertebral disc 3 with a Kirschner wire 14 in an extraforaminal path 23 in front of the processus transversus 6 whereby the use of X-ray control, e.g. an image intensifier is mandatory (Fig. 5);
3. Inserting along the Kirschner wire 14 a trockar 15 and a protection sleeve 16 to the affected intervertebral disc 3 passing the Processus Costalis 17 (Fig. 6);
4. Cutting an access portal 9 of a width W and a height H (Fig. 8) into the Fibrocartilago intervertebralis 18 apart from the anterior longitudinal ligament 5 (Fig. 1) and the posterior longitudinal ligament 4 (Fig. 1) with a cutting instrument 19 (Fig. 7);
5. Removing the disc material with curettes, rongeurs, spoons lead through the protection sleeve 16, preferably with use of an endoscope;
6. Cleaning the endplates of the adjacent upper and lower vertebral body 10;11, particularly by use of specific instruments like water jet or ultrasonic devices, preferably with use of an endoscope;
7. Removing the affected intervertebral disc 3 by slightly bending a distractor 20 in order to keep the view to the situs free for the surgeon, whereby the distraction is performed through the protection sleeve 16 (Fig. 6) or a soft tissue retractor (Fig. 9);
8. Inserting the intervertebral prosthesis 30 through the access portal 9 by means of an implant holder 22 whereby the use of X-ray control, e.g. an image intensifier in order to control the position of the intervertebral prosthesis 30 is mandatory (Fig. 10).

A detailed description of an extraforaminal approach to vertebral bodies in case of posterolateral fusion of vertebrae, e.g. by securing bone grafts to the pedicles or transverse processes by means of bone screws can be found in:
Watkins Melvin B. "Posterolateral fusion of the lumbar and lumbosacral spine" J Bone Joint Surg Am. 1953 Oct;35-A(4):1014-8
Watkins Melvin B. "Posterolateral fusion in pseudoarthrosis and posterior element defects of the lumbosacral spine" Clin Orthop Relat Res. 1964 Jul-Aug;35:80-5.

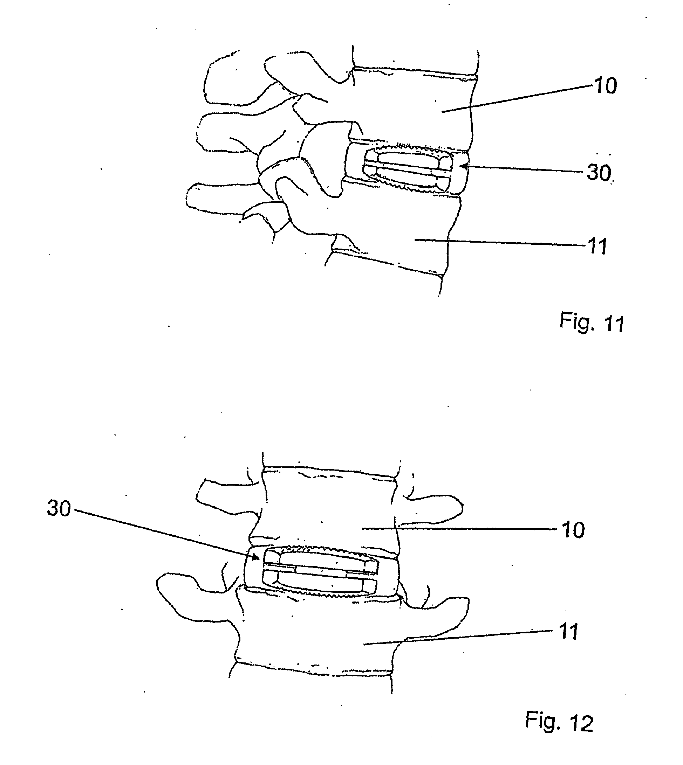

Figs. 11 and 12 illustrate the situation after completion of the surgical process, i.e. an intervertebral prosthesis 30 has been inserted between the two vertebral bodies 10;11. The intervertebral prosthesis 30 shown comprises an upper and a lower apposition member 24;25 the outer surfaces of which abut the end plates of the vertebral bodies 10;11. Furthermore, the intervertebral prosthesis 30 comprises an articulation 26 jointedly connecting the upper and lower apposition member 24;25 such allowing articulating motion between the two apposition members 24;25 and consequently of the two adjoining vertebrae 10;11.

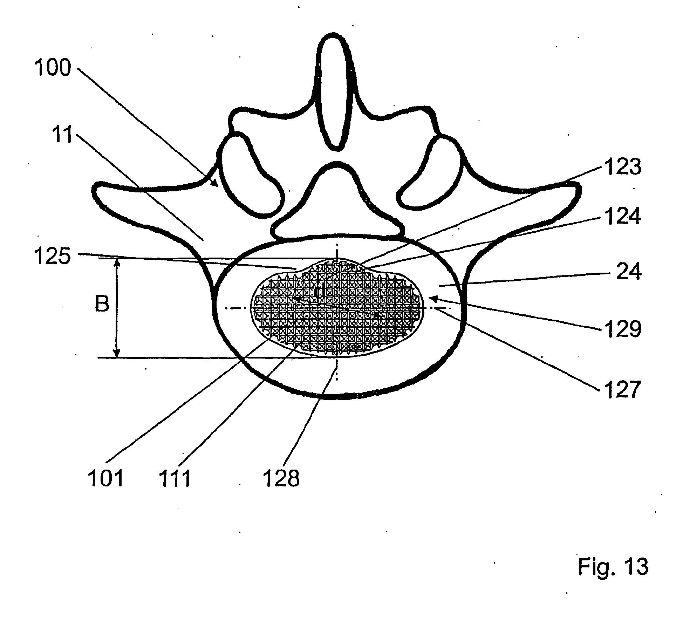

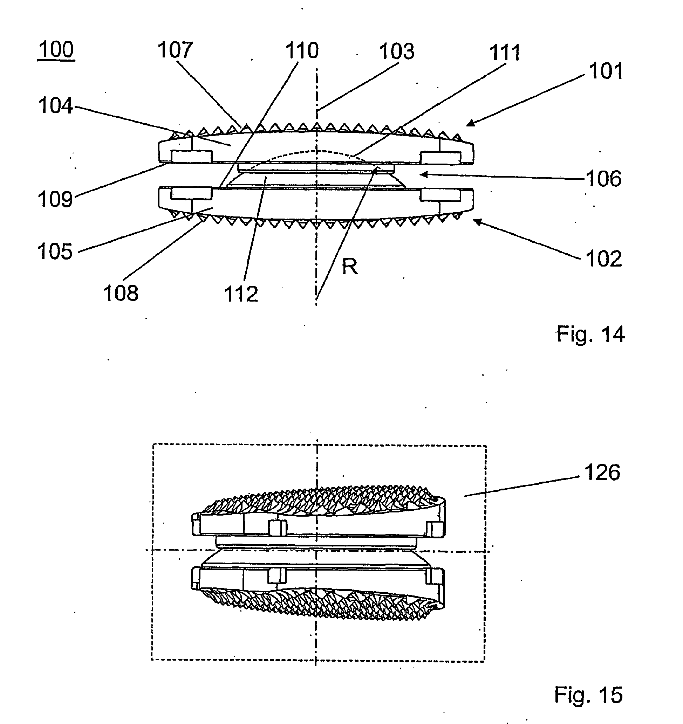

Figs. 13-15 illustrate an embodiment of the intervertebral prosthesis 100 comprising a first prosthetic component 101, a second prosthetic component 102 and an articulation 106 articulatedly connecting said first and second prosthetic component 101;102. Opposite said articulation 106 the first and second prosthetic component 101;102 comprise a first apposition surface 107, respectively a second apposition surface 108, whereby said first apposition surface 107 is configured for abutting the base plate of a first intervertebral body 10 contacting the intervertebral prosthesis 100 on top and said second apposition surface 108 is configured for abutting the cover plate of a second intervertebral body 11 contacting the intervertebral prosthesis 100 at the bottom. The articulation 106 is configured as a ball-and-socket joint, said ball-and-socket joint comprising a spherical cap 112 at the second prosthetic component 102 and congruent to said spherical cap 112 a spherical recess 111 in the first prosthetic component 101.

Each of the first and second apposition surfaces 108; 109 is disposed transversely to the central axis 103. When viewed parallel to said central axis 103 said first and second prosthetic components 101;102 have an elongated shape with a major axis 127 and a transverse minor axis 128, whereby said central axis 103, major axis 127 and transverse minor axis 128 intersect each other. Said central axis 103 and said transverse minor axis 128 further define a middle plane 126. Furthermore, said first and second prosthetic component 101;102 have a cross-sectional area orthogonal to said central axis 103 which is essentially elliptical and comprises two concavities 125 lying on different sides of said middle plane 126 and on the same side of said major axis 127.

The two concavities 125 are disposed symmetrically to said middle plane 126 such that one of said two concavities 125 is arranged in a first quadrant of a circle the centre of which coincides with the point of intersection of the major axis 127, the transverse minor axis 128 and the central axis 103 and the circumference of which is tangent to the periphery 129 of said cross-sectional area at the principal vertices. The second of said two concavities 125 is arranged in a clockwise succeeding, second quadrant of said circle. Furthermore, the two concavities 125 have an essentially semi-elliptical shape and have a depth T measured parallel to said transverse minor axis 128 amounting to about 5% of the maximum width B of said first and second prosthetic components 101;102.

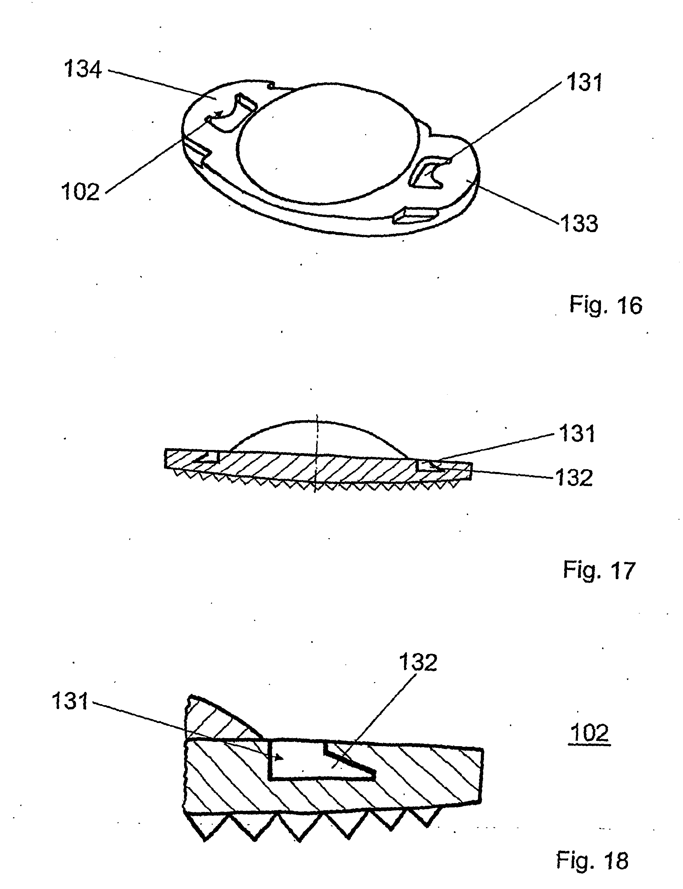

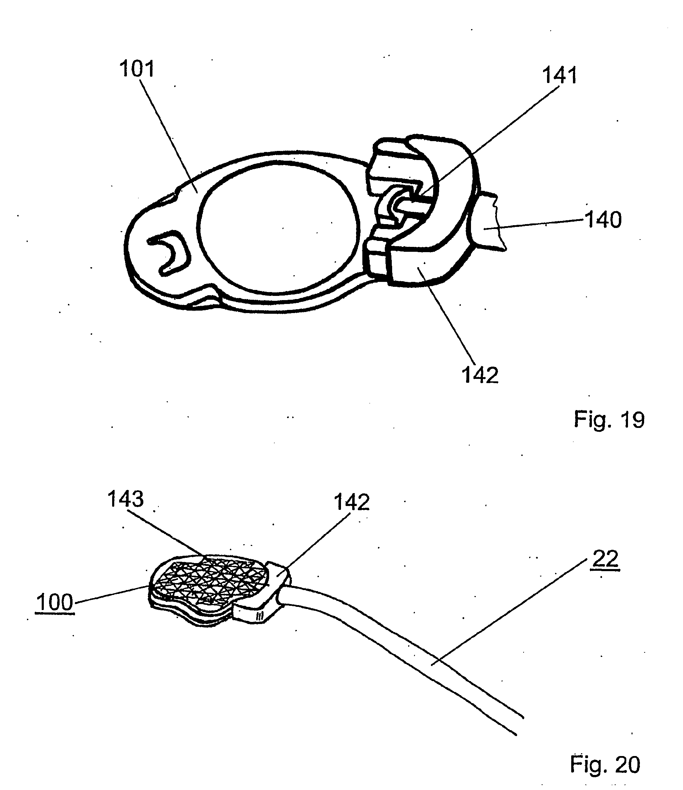

The first and second prosthetic component 101; 102 each comprise an intermediate surface 109; 110, said first and second intermediate surfaces 109;110 facing each other. As shown in figs. 16 - 18 the second intermediate surface 110 is provided with two cavities 131 with an undercut 132 being located at the first and second lateral end 133; 134 of said first and second prosthetic components 101;102 whereby said cavities 131 with undercut 132 are apt for acceptance of an anchoring member 141 of an implant holder 22 (fig. 19,20) whereby the surgeon may select the respective cavity 133 in case of approaching the intervertebral space from the left or from the right side.

Fig. 19 and 20 illustrate an implant holder 22 which is provided with a sleeve 140 and an anchoring member 141, with a hook member at the front end. The anchoring member 141 may be inserted into the cavity 131 and undercut 132 and is displaceably disposed in the central bore of the sleeve 140. The implant holder 22 further has a front end 142 being provided with a fore-part 143 which is adapted to the lateral ends 133,134 (fig. 16) of the first and/or second prosthetic component 101,102. Furthermore, the implant holder 22 is provided with a fastening mechanism (not shown) permitting to pull the anchoring member 141 into the sleeve 140 such pressing the lateral end 133 of the intervertebral prosthesis against the fore-part 143. The sleeve 140 is curved in order to permit an insertion of the intervertebral prosthesis 100 along an extraforaminal path.

## Claims

1. An intervertebral prosthesis (100), in particular an intervertebral disk prosthesis comprising
A) a first prosthetic component (101) having a first apposition surface (107) disposed transversely to the central axis (103) for contacting the end plate of a first adjoining vertebral body;
B) a second prosthetic component (102) having a second apposition surface (108) disposed transversely to the central axis (103) for contacting the end plate of a second adjoining vertebral body; whereby
C) said first and second prosthetic components (101;102) are connected by means of an articulation (106); whereby
D) when viewed parallel to said central axis (103) said first and second prosthetic components (101;102) have an elongated shape with a major axis (127) and a transverse minor axis (128);
E) said central axis (103), major axis (127) and transverse minor axis (128) intersecting each other and said central axis (103) and transverse minor axis (128) defining a middle plane (126);
F) said first and second prosthetic component (101;102) have a cross-sectional area orthogonal to said central axis (103) which is essentially oval or elliptical; **characterised in that**
G) said cross-sectional area comprises at least two concavities (125) lying on different sides of said middle plane (126) and on the same side of said major axis (127).

2. The intervertebral prosthesis (100) as claimed in claim 1, wherein said cross-sectional area of said first and second prosthetic component (101;102) is kidney shaped with an enlargement arranged essentially symmetrical to said middle plane (126).

3. The intervertebral prosthesis (100) as claimed in claim 1 or 2 wherein said at least two concavities (125) have an essentially semi-elliptical or semi-oval shape.

4. The intervertebral prosthesis (100) as claimed in one of the claims 1 to 3, wherein said at least two convavities (125) are disposed essentially symmetrical to said middle plane (126).

5. The intervertebral prosthesis (100) as claimed in one of the claims 1 to 4, wherein said first and second prosthetic component (101;102) have a length H measured parallel to said major axis (127) and wherein each of said at least two concavities (125) has a width W measured parallel to said major axis (127), said width W amounting to between 15% and 35% of said length H.

6. The intervertebral prosthesis (100) as claimed in one of the claims 1 to 5, wherein said first and second prosthetic component (101;102) have a maximum width B measured parallel to said transverse minor axis (128) and wherein each of said at least two concavities (125) has a depth T measured parallel to said transverse minor axis (128), said depth T amounting to between 3% and 25% of said maximum width B.

7. The intervertebral prosthesis (100) as claimed in one of the claims 1 to 6, wherein said cross-sectional area of said first and second prosthetic components (101;102) has an essentially elliptical periphery (129) with a smaller radius of curvature at the first subsidiary vertex than at the second subsidiary vertex of said periphery (129).

8. The intervertebral prosthesis (100) as claimed in claim 7, wherein said first subsidiary vertex is on the same side of said major axis (127) as said at least two concavities (125).

9. The intervertebral prosthesis (100) as claimed in claims 1 or 8, wherein said first and second prosthetic component (101;102) have a length H measured parallel to said major axis (127) and a maximum width B measured parallel to said transverse minor axis (128), whereby the ratio of the length H to the maximum width B is between 3 : 1 and 5:1.

10. The intervertebral prosthesis (100) as claimed in one of the claims 1 to 9, wherein said articulation (106) is configured as a ball-and-socket joint comprising a spherical cap (112) connected to one of the first and second prosthetic components (101;102) and congruent to said spherical cap (112) a spherical recess (111) in the other of said first and second prosthetic components (101;102).

11. The intervertebral prosthesis (100) as claimed in claim 10, wherein one of the first and second prosthetic components (101;102) and the spherical cap (112) consist of one piece.

12. The intervertebral prosthesis (100) as claimed in claim 10, wherein said intervertebral prosthesis (100) is realised in at least three pieces with the spherical cap (112) being the third piece attachable to one of said first and second prosthetic components (101;102).

13. The intervertebral prosthesis (100) as claimed in one of the claims 10 or 12, wherein said intervertebral prosthesis (100) is realised in at least three pieces and comprises an articular shell including the recess (111) which is attachable to one of said first and second prosthetic components (101;102) as a third piece.

14. The intervertebral prosthesis (100) as claimed in claim 13, wherein the spherical cap (112) and the articular shell consist of a material pairing of metal and plastic.

15. The intervertebral prosthesis (100) as claimed in claim 13, wherein the spherical cap (112) and the articular shell consist of a ceramic-to-ceramic material pairing.

16. The intervertebral prosthesis (100) as claimed in one of the claims 1 to 15, wherein the surfaces of the spherical cap (112) and of the recess (111) are coated with titanium carbide or with amorphous carbon (ADLC).

17. The intervertebral prosthesis (100) as claimed in one of the claims 1 to 16, wherein at least the spherical cap (112) is made of a memory metal.

18. The intervertebral prosthesis (100) as claimed in one of the claims 10 to 16, wherein at least the spherical cap (112) is made of a material capable of swelling.

19. The intervertebral prosthesis (100) as claimed in one of the claims 10 to 16, wherein at least the spherical cap (112) is made of a flowable, thermosetting material.

20. The intervertebral prosthesis (100) as claimed in claim 19, wherein at least the spherical cap (112) is made of a monomer, comonomer, homopolymer, oligomer, or mixtures which contains a thermosetting, flowable substance.

21. The intervertebral prosthesis (100) as claimed in claim 20, wherein the thermosetting, flowable substance is selected from the group of:
a) polyethylene glycols, preferably polyethylene glycol(di)-acrylates;
b) n-vinylpyrrolidones; and
c) vinyls, preferably vinyl alcohols; and
d) styrenes.

22. The intervertebral prosthesis (100) as claimed in one of the claims 1 to 21, wherein said first prosthetic component (101) is selected from a first kit of at least M ≥ 2 first prosthetic components (101).

23. The intervertebral prosthesis (100) as claimed in one of the claims 1 to 22, wherein said second prosthetic component (102) is selected from a second kit of at least N ≥ 2 second prosthetic components (102).

24. The intervertebral prosthesis (100) as claimed in one of the claims 1 to 23, wherein said first and second prosthetic component (101;102) each comprise an intermediate surface (109;110), said first and second intermediate surfaces (109;110) facing each other and at least the first intermediate surface (109) being provided with at least one cavity (131) with an undercut (132) being located at the first or/and second lateral end (133;134) of said first and second prosthetic components and being apt for acceptance of an anchoring member (141) of an implant holder (22).

## Patentansprüche

1. Eine Zwischenwirbelprothese (100), insbesondere eine Bandscheibenprothese, umfassend:
A) ein erstes Prothesenelement (101) mit einer ersten quer zur Zentralachse (103) angeordneten Kontaktfläche (107), zur Anlage an die Endplatte eines ersten benachbarten Wirbelkörpers;
B) ein zweites Prothesenelement (102) mit einer zweiten quer zur Zentralachse (103) angeordneten Kontaktfläche (108), zur Anlage an die Endplatte eines zweiten benachbarten Wirbelkörpers; wobei
C) die ersten und zweiten Prothesenelemente (101;102) durch ein Gelenk (106) miteinander verbunden sind; wobei
D) wenn parallel zu Zentralachse (103) betrachtet, die ersten und zweiten Prothesenelemente (101;102) eine longitudinale Form mit einer größeren Achse (127) und einer kleineren Querachse (128) haben;
E) die Zentralachse (103), die größere Achse (127) und die kleinere Querachse (128) sich schneiden, und die Zentralachse (103) und die kleinere Querachse (128) eine Mittelebene (126) bestimmen;
F) die ersten und zweiten Prothesenelemente (101;102) jeweils eine orthogonal zur Zentralachse (103) liegende Querschnittfläche haben, die im Wesentlichen oval oder elliptisch ist, **dadurch gekennzeichnet, dass**
G) die Querschnittfläche mindestens zwei Konkavitäten (125) umfasst, die auf verschiedenen Seiten der Mittelebene (126) und auf derselben Seite der größeren Achse (127) liegen.

2. Zwischenwirbelprothese (100) gemäß Anspruch 1, **dadurch gekennzeichnet, dass** die Querschnittflächen der ersten und zweiten Prothesenelemente (101;102) nierenförmig gestaltet und mit einer Verbreiterung versehen sind, die im Wesentlichen symmetrisch zur Mittelebene (126) angeordnet ist.

3. Zwischenwirbelprothese (100) gemäß Anspruch 1 oder 2, **dadurch gekennzeichnet, dass** die mindestens zwei Konkavitäten (125) eine im Wesentlichen semi-elliptische oder semi-ovale Form haben.

4. Zwischenwirbelprothese (100) gemäß einem der Ansprüche von 1 bis 3, **dadurch gekennzeichnet, dass** die mindestens zwei Konkavitäten (125) im Wesentlichen symmetrisch zur genannten Mittelebene (126) angeordnet sind.

5. Zwischenwirbelprothese (100) gemäß einem der Ansprüche von 1 bis 4, **dadurch gekennzeichnet, dass** die ersten und zweiten Prothesenelemente (101;102), parallel zur größeren Achse (127) gemessen, eine Länge "H" haben und worin jede der mindestens zwei Konkavitäten (125) eine parallel zur größere Achse (127) gemessene Breite "W" hat, wobei die Breite "W" 15% bis 35% der Länge "H" beträgt.

6. Zwischenwirbelprothese (100) gemäß einem der Ansprüche von 1 bis 5, **dadurch gekennzeichnet, dass** die ersten und zweiten Prothesenelemente (101;102) eine parallel zur kleineren Querachse (128) gemessene Höchstbreite "B" aufweisen und jede der mindestens zwei Konkavitäten (125) eine parallel zur kleineren Querachse (128) gemessene Tiefe "T" hat, die 3% bis 25% der Höchstbreite "B" beträgt.

7. Zwischenwirbelprothese (100) gemäß einem der Ansprüche von 1 bis 6, **dadurch gekennzeichnet, dass** die Querschnittfläche der ersten und zweiten Prothesenelemente (101;102) eine im Wesentlichen elliptische Peripherie (129) mit einem am ersten Nebenscheitel kleineren Krümmungsradius als am zweiten Nebenscheitel der Peripherie (129) hat.

8. Zwischenwirbelprothese (100) gemäß Anspruch 7, **dadurch gekennzeichnet, dass** der erste Nebenscheitel auf derselben Seite der größeren Achse (127) wie die mindestens zwei Konkavitäten (125) liegt.

9. Zwischenwirbelprothese (100) gemäß Ansprüchen 1 oder 8, **dadurch gekennzeichnet, dass** die ersten und zweiten Prothesenelemente (101;102) eine parallel zur größeren Achse (127) gemessene Länge "H" und eine parallel zur kleineren Querachse (128) gemessene Höchstbreite "B" haben, wobei das Verhältnis der Länge "H" zur Höchstbreite "B" zwischen 3:1 und 5:1 liegt.

10. Zwischenwirbelprothese (100) gemäß einem der Ansprüche von 1 bis 9, **dadurch gekennzeichnet, dass** das Gelenk (106) als Kugelgelenk ausgebildet ist, das eine mit einem der ersten und zweiten Prothesenelemente (101;102) verbundene sphärische Kalotte (112) und eine zu der sphärische Kalotte (112) kongruente sphärische Vertiefung (111) im anderen der genannten ersten und zweiten Prothesenelemente (101;102) umfasst.

11. Zwischenwirbelprothese (100) gemäß Anspruch 10, **dadurch gekennzeichnet, dass** eines der ersten und zweiten Prothesenelemente (101;102) und die sphärische Kalotte (112) aus einem einzigen Stück bestehen.

12. Zwischenwirbelprothese (100) gemäß Anspruch 10, **dadurch gekennzeichnet, dass** die Zwischenwirbelprothese (100) aus mindestens drei Stücken gefertigt ist, wobei die sphärische Kalotte (112) das dritte, an einem der ersten und zweiten Prothesenelemente (101;102) befestigbare Stück ist.

13. Zwischenwirbelprothese (100) gemäß einem der Ansprüche 10 oder 12, **dadurch gekennzeichnet, dass** die Zwischenwirbelprothese (100) aus mindestens drei Stücken gefertigt ist und eine Gelenkschale mit der Vertiefung (111) umfasst, die als ein drittes Stück an einem der ersten und zweiten Prothesenelemente (101;102) befestigt werden kann.

14. Zwischenwirbelprothese (100) gemäß Anspruch 13, **dadurch gekennzeichnet, dass** die sphärische Kalotte (112) und die Gelenkschale aus einer Materialpaarung von Metall und Kunststoff bestehen.

15. Zwischenwirbelprothese (100) gemäß Anspruch 13, **dadurch gekennzeichnet, dass** die sphärische Kalotte (112) und die Gelenkschale aus einer Materialpaarung von Keramik und Keramik besteht.

16. Zwischenwirbelprothese (100) gemäß einem der Ansprüche von 1 bis 15, **dadurch gekennzeichnet, dass** die Oberflächen der sphärischen Kalotte (112) und der Vertiefung (111) mit Titancarbid oder amorphem Kohlenstoff (ADLC) beschichtet sind.

17. Zwischenwirbelprothese (100) gemäß einem der Ansprüche von 1 bis 16, **dadurch gekennzeichnet, dass** mindestens die sphärische Kalotte (112) aus einem Gedächtnis-Metall gefertigt ist.

18. Zwischenwirbelprothese (100) gemäß einem der Ansprüche von 1 bis 16, **dadurch gekennzeichnet, dass** mindestens die sphärische Kalotte (112) aus einem schwellfähigen Material gefertigt ist.

19. Zwischenwirbelprothese (100) gemäß einem der Ansprüche von 1 bis 16, **dadurch gekennzeichnet, dass** mindestens die sphärische Kalotte (112) aus einem fließfähigen, wärmehärtendem Material besteht.

20. Zwischenwirbelprothese (100) gemäß Anspruch 19, **dadurch gekennzeichnet, dass** mindestens die sphärische Kalotte (112) aus einem Monomer, Komonomer, Homopolymer, Oligomer oder Mischungen gefertigt ist, die eine wärmehärtbare, fließfähige Substanz enthalten.

21. Zwischenwirbelprothese (100) gemäß Anspruch 20, **dadurch gekennzeichnet, dass** die wärmehärtbare, fließende Substanz aus der folgenden Gruppe gewählt ist:
a) Polyäthylenglykole, vorzugsweise Polyäthylenglykol-(bi)-Acrylaten;
b) N-Vinylpyrrolidone; und
c) Vinyle, vorzugsweise Vinyl-Alkohole; und
d) Styrene.

22. Zwischenwirbelprothese (100) gemäß einem der Ansprüche von 1 bis 21, **dadurch gekennzeichnet, dass** das erste Prothesenelement (101) aus einem ersten Satz von mindestens M ≥ 2 ersten Prothesenelementen (101) ausgewählt ist.

23. Zwischenwirbelprothese (100) gemäß einem der Ansprüche von 1 bis 22, **dadurch gekennzeichnet, dass** das zweite Prothesenelement (102) aus einem zweiten Satz von mindestens N ≥ 2 zweiten Prothesenelementen (102) ausgewählt ist.

24. Zwischenwirbelprothese (100) gemäß einem der Ansprüche von 1 bis 23, **dadurch gekennzeichnet, dass** jedes der ersten und zweiten Prothesenelemente (101;102) eine Zwischenfläche (109;110) umfasst, wobei die genannten ersten und zweiten Zwischenflächen (109;110) einander gegenüberstehen und mindestens die erste Zwischenfläche (109) mit mindestens einer Kavität (131) mit einer Unterschneidung (132) versehen ist, die am ersten und/oder zweiten seitlichen Ende (133;134) der ersten und zweiten Prothesenelemente angeordnet und für die Aufnahme eines Verankerungsglieds (141) eines Implantat-Halters (22) geeignet ist.

## Revendications

1. Prothèse intervertébrale (100), en particulier prothèse de disque intervertébral comprenant
A) un premier élément de prothèse (101) présentant une première surface d'apposition (107) disposée transversalement à l'axe central (103) pour être en contact avec la plaque d'extrémité d'un premier corps vertébral adjacent ;
B) un second élément de prothèse (102) présentant une seconde surface d'apposition (108) disposée transversalement à l'axe central (103) pour être en contact avec la plaque d'extrémité d'un second corps vertébral adjacent ; de sorte que
C) lesdits premier et second éléments de prothèse (101 ; 102) sont raccordés au moyen d'une articulation (106) ; de sorte que
D) lorsqu'observés parallèlement au dit axe central (103), lesdits premier et second éléments de prothèse (101 ; 102) présentent une configuration de forme allongée avec un grand axe (127) et un petit axe transversal (128) ;
E) lesdits axe central (103), grand axe (127) et petit axe transversal (108) s'entrecoupant l'un l'autre et ledit axe central (103) et ledit petit axe transversal (128) définissant un plan médian (126) ;
F) lesdits premier et second éléments de prothèse (101 ; 102) présentent une surface de section transversale perpendiculaire au dit axe central (103) qui est essentiellement ovale ou elliptique ;
**caractérisée en ce que**
G) ladite surface de section transversale comporte au moins deux concavités (125) reposant sur des côtés différents dudit plan médian (126) et sur le même côté dudit grand axe (127).

2. Prothèse intervertébrale (100) selon la revendication 1, dans laquelle ladite surface de section transversale desdits premier et second éléments de prothèse (101 ; 102) est configurée en forme de rein avec une partie élargie disposée de façon essentiellement symétrique par rapport au dit plan médian (126).

3. Prothèse intervertébrale (100) selon la revendication 1 ou 2 dans laquelle lesdites au moins deux concavités (125) présentent une forme essentiellement semi-elliptique ou semi-ovale.

4. Prothèse intervertébrale (100) selon l'une des revendications 1 à 3, dans laquelle lesdites au moins deux concavités (125) sont disposées de façon essentiellement symétrique par rapport au dit plan médian (126).

5. Prothèse intervertébrale (100) selon l'une des revendications 1 à 4, dans laquelle lesdits premier et second éléments de prothèse (101 ; 102) présentent une longueur H mesurée parallèlement au dit grand axe (127) et dans laquelle chacune des dites au moins deux concavités (125) présente une largeur (W) mesurée parallèlement au dit grand axe (127), ladite largeur (W) représentant entre 15% et 35% de ladite longueur H.

6. Prothèse intervertébrale (100) selon l'une des revendications 1 à 5, dans laquelle lesdits premier et second éléments de prothèse (101 ; 102) présentent une largeur maximale B mesurée parallèlement au dit petit axe transversal (128) et dans laquelle chacune des dites au moins deux concavités (125) présente une profondeur T mesurée parallèlement au dit petit axe transversal (128), ladite profondeur T représentant entre 3% et 25% de ladite largeur maximale B.

7. Prothèse intervertébrale (100) selon l'une des revendications 1 à 6, dans laquelle ladite surface de section transversale desdits premier et second éléments de prothèse (101 ; 102) présente une périphérie essentiellement elliptique (129) avec un rayon de courbure plus petit au niveau du premier sommet supplémentaire qu'au niveau du second sommet supplémentaire de ladite périphérie (129).

8. Prothèse intervertébrale (100) selon la revendication 7, dans laquelle ledit premier sommet supplémentaire est sur le même côté dudit grand axe (127) que lesdites au moins deux cavités (125).

9. Prothèse intervertébrale (100) selon les revendications 1 ou 8, dans laquelle lesdits premier et second éléments de prothèse (101 ; 102) présentent une longueur H mesurée parallèlement au dit grand axe (127) et une largeur maximale B mesurée parallèlement au dit petit axe transversal (128), de sorte que le rapport de la longueur H à la largeur maximale B se situe entre 3:1 et 5:1.

10. Prothèse intervertébrale (100) selon l'une des revendications 1 à 9, dans laquelle ladite articulation (106) est configurée sous la forme d'un emboîtement réciproque comportant une tête sphérique (112) raccordée à l'un des premier et second éléments de prothèse (101 ; 102) et coïncidant avec ladite tête sphérique (112), un évidement sphérique (111) dans l'autre desdits premier et second éléments de prothèse (101 ; 102).

11. Prothèse intervertébrale (100) selon la revendication 10, dans laquelle l'un des premier et second éléments de prothèse (101 ; 102) et tête sphérique (112) sont constitués d'une seule pièce.

12. Prothèse intervertébrale (100) selon la revendication 10, dans laquelle ladite prothèse intervertébrale (100) est réalisée en au moins trois morceaux, la tête sphérique (112) étant la troisième pièce qui peut être fixée à l'un desdits premier et second éléments de prothèse (101 ; 102).

13. Prothèse intervertébrale (100) selon l'une des revendications 10 ou 12, dans laquelle ladite prothèse intervertébrale (100) est réalisée en au moins trois morceaux et comporte une coque articulaire incluant l'évidement (111) laquelle peut être fixée à l'un desdits premier et second éléments de prothèse (101 ; 102) comme un troisième morceau.

14. Prothèse intervertébrale (100) selon la revendication 13, dans laquelle la tête sphérique (112) et la coque articulaire sont constituées d'une paire de matériaux de métal et de matière plastique.

15. Prothèse intervertébrale (100) selon la revendication 13, dans laquelle la tête sphérique (112) et la coque articulaire sont constituées d'une paire de matériaux céramique-céramique.

16. Prothèse intervertébrale (100) selon l'une des revendications 1 à 15, dans laquelle les surfaces de la tête sphérique (112) et de l'évidement (111) sont recouvertes de carbure de titane ou de carbone amorphe (ADLC).

17. Prothèse intervertébrale (100) selon l'une des revendications 1 à 16, dans laquelle au moins la tête sphérique (112) est constituée d'un alliage à mémoire.

18. Prothèse intervertébrale (100) selon l'une des revendications 10 à 16, dans laquelle au moins la tête sphérique (112) est constituée d'un matériau capable de gonfler.

19. Prothèse intervertébrale (100) selon l'une des revendications 10 à 16, dans laquelle au moins la tête sphérique (112) est constituée d'un matériau fluidifiable, thermodurcissable.

20. Prothèse intervertébrale (100) selon la revendication 19, dans laquelle au moins la tête sphérique (112) est constituée d'un monomère, d'un co-monomère, d'un homopolymère, d'un oligomère, ou de mélanges qui contiennent une substance fluidifiable, thermodurcissable.

21. Prothèse intervertébrale (100) selon la revendication 20, dans laquelle la substance fluidifiable, thermodurcissable est sélectionnée à partir du groupe constitué de :
a) polyéthylène glycols, de préférence de
polyéthylène glycol(di)-acrylates ;
b) n-vinylpyrrolidone ; et
c) vinyles, de préférence d'alcools de vinyl ; et
d) styrènes.

22. Prothèse intervertébrale (100) selon l'une des revendications 1 à 21, dans laquelle ledit premier élément de prothèse (101) est sélectionné à partir d'un premier ensemble constitué d'au moins M ≥ 2 premiers éléments de prothèse (101).

23. Prothèse intervertébrale (100) selon l'une des revendications 1 à 22, dans laquelle ledit second élément de prothèse (102) est sélectionné à partir d'un second ensemble constitué d'au moins N ≥ 2 seconds éléments de prothèse (102).

24. Prothèse intervertébrale (100) selon l'une des revendications 1 à 23, dans laquelle lesdits premier et second éléments de prothèse (101, 102) comprennent, chacun, une surface intermédiaire (109 ; 110), lesdites première et seconde surfaces intermédiaires (109 ; 110) se faisant face l'une l'autre et la première surface intermédiaire au moins (109) étant dotée d'au moins une cavité (131) présentant une découpe (132) placée au niveau de la première ou/et de la seconde extrémité latérale (133 ; 134) desdits premier et second éléments de prothèse et étant adaptée pour recevoir un élément d'ancrage (141) d'un support d'implant (22).
